# EUROPEAN PATENT APPLICATION

(11) **EP 1 792 612 A2**
(43) Date of publication of application: **06.06.2007**
(21) Application number: 06125101.3
(22) Date of filing: 30.11.2006
(51) Int. Cl.: A61K 9/70, A23L 1/29

(54) **Transdermal nutritional supplement delivery patch**

(30) Priority: 30.11.2005 US 290279
(71) Applicant: Nexagen USA LLC, Akron OH 44319 (US)
(72) Inventor: Crawford, David S., Akron, OH 44312 (US)
(74) Representative: Patentanwälte Freischem

(57) **Abstract**

The present invention discloses a patch containing a transdcrmally diffusible nutritional supplement formulated for the treatment of health conditions, including, weight loss. The patch may be comprised of a fabric layer on which is deposited the nutritional supplement, an adhesive and means for protecting the adhesive. The nutritional supplement may be a mixture of two or more of forskohlin, epicathechin, proanthocyanidins, caffeine, chromium, 5-hydroxytryptophan, diiodotyrosine, and select permeation enhancers, which may include piperine or tetrahydropiperine. Methods for using transdermal nutritional supplement delivery patches are also taught.

## Description

### I. Background of the Invention

### A. Field of Invention

This invention pertains to the art of methods and apparatuses for the transdermal delivery of nutritional supplements by means of an infused patch and methods for promoting weight loss using patches infused with blends of selected nutritional supplements.

### B. Description of the Related Art

Nutritional supplements have become a common alternative to more traditional drug-related therapies for improving and maintaining health and treating adverse health conditions and their associated symptoms. Defined as a food or part of a food that may provide medicinal or health benefits, including the prevention and treatment of disease, nutritional supplements include such things as vitamins, minerals, amino-acids, antioxidants, herbal and botanical extracts and other dietary supplements that may offer nutritional and, in some cases, therapeutic value to associated users.

Nutritional supplements have been promoted for improving a wide variety of health conditions, including stress and obesity, and symptoms related to health conditions, such as pain related to arthritis. The wide-spread use of nutritional supplements reflects a genuine interest on the part of consumers to find alternative treatments to health conditions that affect them. Many consumers are wary of the side effects of traditional drug therapies and many more consumers desire to improve their health even in the absence of a definable illness, using nutritional supplements to, among other things, increase energy, decrease stress and improve metabolism.

Among the principal thrusts of the nutritional supplements industry have been the treatment of obesity and the creation of supplement blends directed toward promoting weight loss. Obesity is a significant, growing public health problem in the United States. Radical weight loss methods, including surgical options and extreme diets may have side effects that are undesirable and discourage people from taking steps to lose weight. While it is widely accepted that a healthy diet combined with exercise is a highly effective way to lose weight, some individuals get discouraged when weight loss is not as rapid as anticipated or they simply do not want to undertake diet or exercise. The use of certain nutritional supplements and combinations of the same, used by themselves or in conjunction with a proper diet and exercise, may improve weight loss and also improve energy. In the past, supplemental diet products containing ephedra were widely used in relation to weight loss and increasing energy, but many of these products proved to be unsafe. A wide variety of other non-ephedra containing supplements for improving weight loss and energy have since entered the market. These products are typically ingested orally, either in a liquid or pill form, or as supplements to other foods. Oral ingestion may result in reduced uptake of the nutritionals and, accordingly, reduced benefit. Moreover, many individuals have difficulty swallowing pills, which makes the administration of nutritional supplements difficult.

An alternative to oral ingestion is transdermal delivery of nutritional supplements. Such a delivery method avoids the problems associated with oral administration of these elements, while offering an effective method for introducing nutritional supplements directly into the bloodstream through the skin. Accordingly, it is the object of the present invention to provide a means of administering nutritional supplements transdermally, and more specifically, for introducing, transdermally, nutritional supplements and blends thereof, directed at promoting weight loss.

### II. Summary of the Invention

According to one aspect of the invention, a device for transdermally administering a dose of nutritional supplements is provided.

According to another aspect of the invention, a patch infused with a nutritional supplement blend have the property of being diffusible through an associated user's skin is provided.

According to yet another aspect of the invention, the patch includes a fabric layer, an adhesive layer, and a release liner.

According to still another aspect of the invention, the patch is infused with a nutritional supplement blend selected for the promotion of weight loss.

According to a further aspect of the invention, the patch is infused with a nutritional supplement blend selected to promote weight loss and increase energy.

According to another aspect of the invention, the patch is infused with a nutritional supplement blend selected to promote weight loss, increase energy, and containing antioxidants.

According to still another aspect of the invention, the patch is infused with a permeation enhancer selected to enhance permeation of the nutritional supplements through the skin.

According to yet another aspect of the invention, there is provided a nutritional supplement blend selected for the promotion of weight loss, comprising one or more of a supplement selected to suppress appetite, a supplement selected to increase energy, a supplement selected to support pancreatic function, a supplement selected to support dopamine production, a supplement selected to support thyroid function, a supplement selected to support preservation of lean body mass during dieting, and an antioxidant.

According to another aspect of the invention, the nutritional supplement blend is diffusible through the skin of an associated user.

According to another aspect of the invention, the nutritional supplement blend further includes a permeation enhancer selected to enhance permeation of the nutritional supplements through the skin.

According to yet another aspect of the invention, there is provided a nutritional supplement blend comprising one or more of forskohlin, caffeine, chromium, 5-hydroxytryptophan, and diiodotyrosine.

According to another aspect of the invention, a method of promoting weight loss using a patch is taught.

According to yet another aspect of the invention, the method further includes transdermally administering a dose of nutritional supplements.

Still other benefits and advantages of the invention will become apparent to those skilled in the art to which it pertains upon a reading and understanding of the following detailed specification.

### III. Brief Description of the Drawings

The invention may take physical form in certain parts and arrangement of parts, a preferred embodiment of which will be described in detail in this specification and illustrated in the accompanying drawings which form a part hereof and wherein:

FIGURE 1 is a depiction of a patch in accordance with the present invention.

FIGURE 2 is a depiction of the transdermal diffusion of the nutritional supplement from the patch to the associated user.

FIGURE 3 is a depiction of the various locations a patch may be worn.

### IV. Description of the Preferred Embodiment

Referring now to the drawings wherein the showings are for purposes of illustrating a preferred embodiment of the invention only and not for purposes of limiting the same.

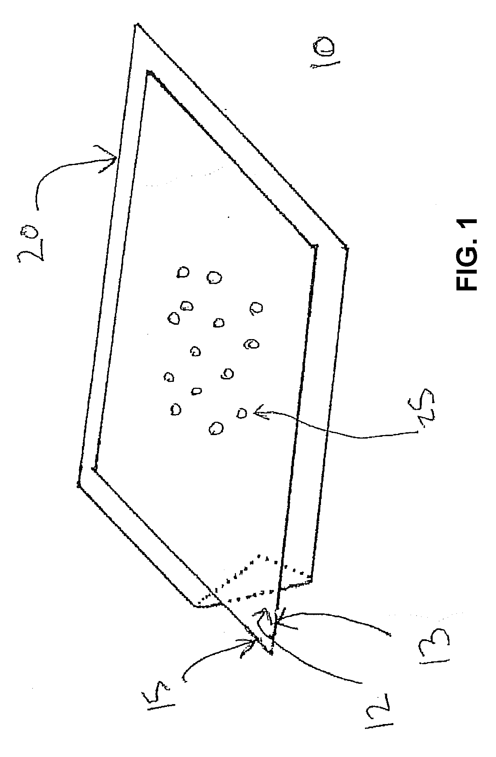

FIGURE 1 shows a patch 10 in accordance with the present invention, for the transdermal delivery of nutritional supplements. The term "transdermal" means "through or by way of the skin." The patch 10 may be a comprised of a fabric layer 15, having an inner surface and an outer surface, and a release liner 20. The inner surface of the fabric layer 15 may be the surface that is placed nearest the associated user's skin.

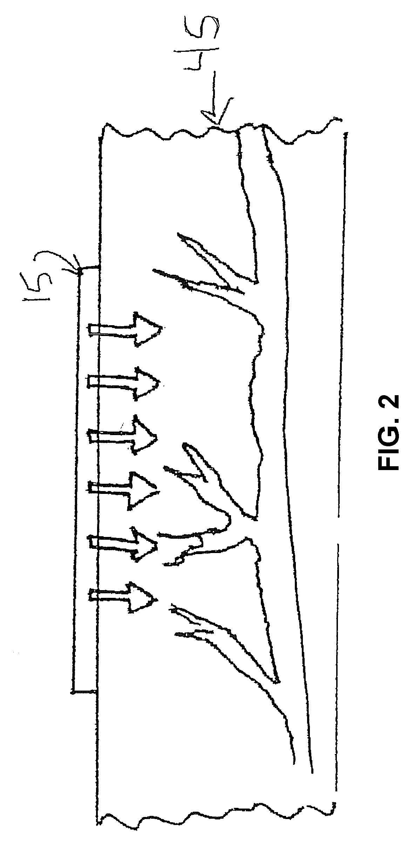

The fabric layer 15 may be the substrate for the nutritional supplement 25 being administered. In using the term "substrate", it is meant that the fabric layer 15 is the layer of the patch 10 on which the nutritional supplement 25 is stored prior to the transdermal delivery of the nutritional supplement 25 to the associated user (as shown in FIGURE 2), through the associated user's skin. The nutritional supplement 25 may be applied substantially to the surface of the fabric layer 15 by methods that are well known in the art. Alternatively, the nutritional supplement 25 may impregnate the fabric layer 15. In this respect, the fabric layer 15 may be absorbent of the nutritional supplement 25. For purposes of this invention, the nutritional supplement 25 is "deposited on" the fabric layer 15 whether it is deposited substantially on the inner surface of the fabric layer 15 or is absorbed by the fabric layer 15.

The outer surface 13 of the fabric layer 15, namely the surface that is intended to be furthest from contact with the skin of the associated user, may be substantially impermeable to the nutritional supplement 25. Alternatively, there may be provided a separate impermeable layer affixed to the outer surface 13 of the fabric layer 15. In this way, the nutritional supplement 25 will not soak through or evaporate through the outer surface 13 of the fabric layer 15.

The fabric layer 15 may be a polyester layer, which may be a foam or woven on non-woven polyester fiber. Alternatively, the fabric layer 15 may be comprised of polyethylene, vinyl, cotton, polyolefin or any other natural or man-made material, or blends of the aforementioned materials, selected with sound engineering judgment. As noted above, the fabric layer 15 may be absorbent to the nutritional supplement 25. The fabric layer 15 may alternatively be a non-absorbent material on which the nutritional supplement 25 is deposited. In one embodiment, the fabric layer 15 may be comprised of a polyester fabric sold under the tradename Volera 60E skintone supplied by Voltec (Sansui).

The dimensions of the fabric layer 15 may be selected to provide a suitable area of contact with the associated user's skin to supply a desired dosage of the nutritional supplement 25 to the associated user. In one embodiment, the fabric layer may have a length of between about 0.5 inches and about 2.5 inches, and a width of between about 0.5 inches and about 2.5 inches. It will be immediately understood, however, that the dimensions of the fabric layer, as with its shape, may vary, having a width and length less than 0.5 inches or greater than 2.5 inches.

Whether the nutritional supplement 25 is impregnated into the fabric layer 15 or deposited onto the inner surface of the fabric layer 15, there may also be provided in association with the fabric layer 15 an adhesive (not shown). The adhesive may be a compound which allows for removably affixing the fabric layer 15 to the skin 45 of an associated user. The adhesive is preferably selected to be non-reactive with the nutritional supplement 25. Adhesives used in transdermal patch systems are well known in the art and may include silicones and polyisobutylenes. Pressure sensitive adhesives, such as acrylates, polyisobutylene/light mineral oil blends, polystryene-polybutadiene block copolymer/mineral oil blends, may be used in accordance with the present invention. In one embodiment, the adhesive may be that which is sold under the tradename Solutia Gelva 737. The adhesive may be blended with the nutritional supplement 25 before being deposited on or into the fabric layer 15; alternatively, the adhesive may be deposited onto the fabric layer 25 before or after the nutritional supplement 25 is deposited. It should be noted that the adhesive may be permeable to the nutritional supplement 25 so that the nutritional supplement can pass through the adhesive layer and into the skin 45.

The nutritional supplement 25, may include one or a blend of more than one supplement having the property of being able to diffuse through the skin of an associated user, either or both with or without a permeation enhancer (discussed below). Supplements selected in accordance with the present invention may include vitamins, minerals, amino-acids, antioxidants, herbal extracts, and botanical extracts. The nutritional supplement 25 may be comprised of one or more supplements that alone, or in combination, are selected to promote the treatment a particular heath condition. Alternatively, the nutritional supplement 25 may be comprised of one or more supplements that alone, or in combination, are selected to promote the treatment of more than one heath condition. In one embodiment, the nutritional supplement 25 may be selected to promote weight loss. In an another embodiment, the nutritional supplement 25 may be selected to promote weight loss and to treat symptoms believed to be associated with weight loss and dieting, including, feelings of lowered energy, depressed emotions, and reduction of select organ functionality, including the pancreas and thyroid.

In one embodiment, which may relate to a patch for promoting weight loss and to treat symptoms believed to be associated with weight loss and dieting, the nutritional supplement 25 may be a comprised of one or a mixture or more than one of a supplement selected to suppress appetite, a supplement selected to increase energy, a supplement selected to support pancreatic function, a supplement selected to support dopamine production, a supplement selected to support thyroid function, a supplement selected to support preservation of lean body mass during reduced caloric intake, and an antioxidant. It will be noted that some supplements may support more than one of the above functions. It will also be noted that supplements selected in accordance with the present invention preferably have the property of being diffusible through the skin of an associated user either or both with or without a permeation enhancer.

In accordance with the above described embodiment, supplements selected to suppress appetite may include 5-hydroxytryptophan and extracts containing 5-hydroxytryptophan. Other suitable supplements in this category may include hoodia gordonii, sida cordifolia, and red clover, and extracts derived therefrom. 5-hydroxytryptophan may also be selected as a supplement to support dopamine production. It is believed that supporting the production of dopamine in individuals will improve their mood.

Supplements selected to increase energy may include methylxanthines, including caffeine and extracts thereof. Supplements selected to support pancreatic function may include chromium and chromium containing compounds, including chromium analogs. It is believed that chromium assists pancreatic function and, relatedly, increases insulin sensitivity. Supplements selected to support thyroid function may include diiodotyrosine and extracts containing diiodotyrosine, forskohlin and extracts containing forskohlin. It is believed that during dieting, the body slows down production of the thyroid gland's hormones T3 and T4 and a body undergoing reduced calorie intake may start to use muscle glycogen as a source of energy. Diiodotyrosine is a building block for T3 and T4 and it is believed that supplementation can assist the thyroid gland to maintain its function and help preserve lean muscle. Forskohlin is also believed to stimulate the thyroid. Other suitable supplements in this category may include thyroid extracts from porcine or bovine sources.

Suitable antioxidants may include polyphenyls, proanthocyanidins, epicathechin, carnosine, carotenoids, co-enzyme Q10, zinc, selenium, and extracts and blends of the above. It is believed that antioxidants neutralize or deactivate free radicals produced generated as a product of lipid metabolism from weight reduction.
In one embodiment, the nutritional supplement may comprise a mixture of a forskohlin supplement, a caffeine supplement, and a chromium supplement. Forskohlin may be obtained from the herb Coleus forskohlii or extracts of the same, which include, but are not limited to an extract sold under the tradename ForsLean available from Sabinsa Corporation. Chromium may be obtained from or used in the form of chromium polynicotinate, which is sold under the tradename ChromeMate, available from Interhealth. Caffeine may be obtained from the herb Guarana (Paullina cupana) or extracts of the same. It will be noted that the use of Guarana or extracts of the same may provide a source of antioxidants, including epicathechin and proanthocyanidins.

In another embodiment, the nutritional supplement may further include one or more of a 5-hydroxytryptophan supplement and a diiodotyrosine supplement. 5-hydroxytryptophan may be obtained from the plant Griffonia simplicifolia and extracts of the same.

It should be noted that there may be many other sources for the above referenced supplements and any such source, or derivative or extract of the same may be used in accordance with the present invention provided the source provides a suitable dose of the selected supplement and is diffusible through the skin of the associated user either or both with or without a permeation enhancer. The above identified supplement sources are not intended to limit the disclosure of this invention and are exemplary only.

The nutritional supplement 25 may include a sufficient amount of a forskohlin supplement to provide an effective amount of forskohlin to cause a desired dose of forskohlin to diffuse through the skin of the associated user in a selected time period. The effective amount of forskohlin in the nutritional supplement may be from about 2 mg to about 20 mg.

The nutritional supplement 25 may include a sufficient amount of an epicathechin supplement to provide an effective amount of epicathechin to cause a desired dose of epicathechin to diffuse through the skin of the associated user in a selected time period. The effective amount of epicathechin may be from about 1 mg to about 10 mg.

The nutritional supplement 25 may include a sufficient amount of a proanthocyanidins supplement to provide an effective amount of proanthocyanidins to cause a desired dose of proanthocyanidins to diffuse through the skin of the associated user in a selected time period. The effective amount of proanthocyanidins may be from about 1 mg to about 10 mg.

The nutritional supplement 25 may include a sufficient amount of a caffeine supplement to provide an effective amount of caffeine to cause a desired dose of caffeine to diffuse through the skin of the associated user in a selected time period. The effective amount of caffeine may be from about 1 mg to about 40 mg.

The nutritional supplement 25 may include a sufficient amount of a chromium supplement to provide an effective amount of chromium to cause a desired dose of chromium to diffuse through the skin of the associated user in a selected time period. The effective amount of chromium may be from about 50 µg to about 400 µg.

The nutritional supplement 25 may include a sufficient amount of a 5-hydroxytryptophan supplement to provide an effective amount of 5-hydroxytryptophan to cause a desired dose of 5-hydroxytryptophan to diffuse through the skin of the associated user in a selected time period. The effective amount of 5-hydroxytryptophan may be from about 10 mg to about 50 mg.

The nutritional supplement 25 may include a sufficient amount of a diiodotyrosine supplement to provide an effective amount of diiodotyrosine to cause a desired dose of diiodotyrosine to diffuse through the skin of the associated user in a selected time period. The effective amount of diiodotyrosine may be from about 50 µg to about 400 µg.

The nutritional supplement 25 may include an effective amount of one or a blend of more than one permeation enhancer. Permeation enhancers may be used to aid in the passage of the nutritional supplement through the skin. The term "effective amount" with respect to the permeation enhancer refers to the amount appropriate to cause the diffusion of a selected dose of nutritional supplement 25 through the user's skin within a selected period of time.

Many permeation enhancers are known in the art and may include straight-chain or branched-chain fatty alcohols selected from the group consisting of decanol, dodecanol, 2-hexyl decanol, 2-octyl dodecanol and oleyl alcohol; saturated or unsaturated fatty acids selected from the group consisting of undecylenic acid, lauric acid, myristic acid and oleic acid; fatty acid esters or of their derivatives; fatty alcohol ethoxylates selected from the reaction products of dodecanol or oleyl alcohol; methyl laurate, ethyl oleate, isopropyl myristate and isopropyl palmitate; water-miscible enhancer selected from the group consisting of 1,2-propylene glycol, glycerol, 1,3-butanediol, dipropylene glycol and polyethylene glycols. Other compounds known to improve skin permeability to the nutritional supplement 25 may also be included in the patch. Such compounds may include piperine or tetrahydropiperine. Piperine and tetrahydropiperine may be obtained from black pepper or long pepper and extracts thereof. One source of tetrahydropiperine may be that which is sold under the tradename Cosmoperine, available from Sabinsa Corporation.

The effective amount of permeation enhancer may vary according to the blend of the nutritional supplement 25, the desired dose, the area of the patch, or the desired rate of supplement 25 diffusion. In one embodiment, the effective amount of tetrohydropiperine may be from about 0.1 mg to about 2.0 mg.

In one embodiment, the release liner 20 may be a mylar liner. The release liner 20 may be removably affixed to the fabric layer 15. The release liner 20 may be removably affixed to the inner surface 12 of the fabric layer 15, namely the surface that is nearest the skin of the associated user. The release liner 20 may protect the adhesive from contamination, which could prevent the adhesive from adhering the patch 10 to the skin 45 of an associated user. The release liner 20 may also protect the nutritional supplement 25 from contamination. In this respect, the release liner 20 may be substantially impermeable. The release liner 20 may be coated in silicon or other similar materials to aid in the release of the release liner from the fabric layer 15. It should be understood that the release liner 20 may be made of any material that will allow for the release liner 20 to be removably affixed to the fabric layer 15 even in the presence of the adhesive. It should also be understood that there could be at a second release liner (not shown), wherein the first release liner 20 is removably affixed to the inner surface 12 of the fabric layer 15 and the second release liner is removably affixed to the outer surface 13 of the fabric layer 15. Other means for protecting the adhesive and nutritional supplement 25 of the fabric layer 15 may be employed, including, but not limited to encapsulating the fabric layer 15 in a sealed pouch (not shown). In this respect, a variety of other methods for protecting the adhesive from contamination may be selected with sound engineering judgment.

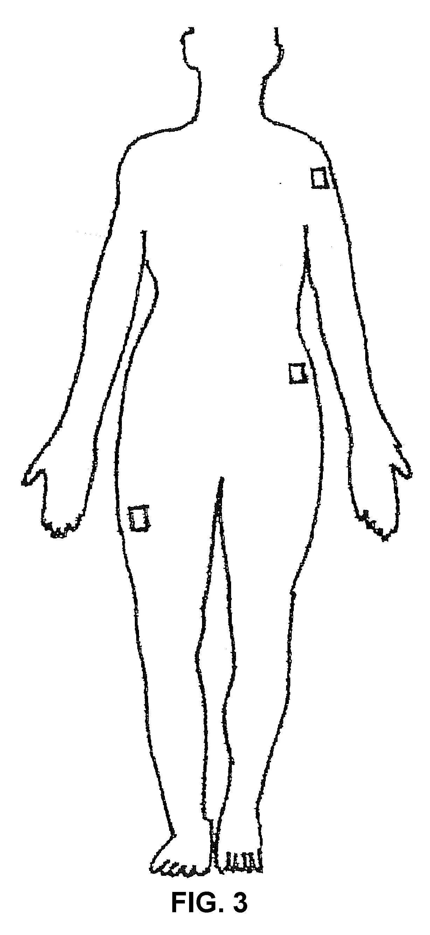

The method of using the patch 10 disclosed herein may involve separating the fabric layer 15 from the release liner 20, or otherwise releasing the fabric layer 15 from whatever means are selected to protect the adhesive from contamination, so as to expose the adhesive. The inner surface 12 of the fabric layer 15 may be placed on the skin 45 of the associated user (as shown in Figure 2), wherein the adhesive may hold the fabric layer 15 in place. Nutritional supplement 25, which may be deposited on the inner surface 12 of the fabric layer 15 or impregnated in the fabric layer 15 may pass through the skin 45 of the associated user, with or without the assistance of a permeation enhancer or other similar compound. The fabric layer 15 may be worn by the associated user for a period of time. The period of time may depend on the dose of supplement to be given, the location on the associated user where the fabric layer 15 is placed, the specific physical characteristics of the associated user, the purpose for which the associated user is taking the nutritional supplement 25, or the characteristics of the nutritional supplement 25. It should be understood that a variety of other factors may affect the period of time the fabric layer 15 is to remain on the skin 45 of the associated user. FIGURE 3 demonstrates some of the variety of locations where the fabric layer 15 may be worn.

In one embodiment, the patch 10 may be a weight loss patch having a nutritional supplement 25 formulated for promoting the reduction of weight in an associated user. In this embodiment, a first patch 10, or more specifically, a first fabric layer 15 removed from the release liner 20, may be placed on the skin 45 of an associated user and be allowed to remain for a first time interval. After the first time interval has expired, the first patch 10 may be removed and replaced with a second patch 10, which may remain in place for a second time interval. The first time interval and the second time interval may be substantially the same time intervals. This process may be repeated until desired weight loss has been reached. Moreover, use of the patch 10 in accordance with this embodiment may be combined with a regimen including either or both exercise and diet.

The use of release liners 20 as disclosed about as disclosed above allow for the long term storage of the fabric layer 15. In this way, the patch 10 can be manufactured for use at a significantly later period of time.

At least one embodiment has been described, hereinabove. It will be apparent to those skilled in the art that the above methods may incorporate changes and modifications without departing from the general scope of this invention. It is intended to include all such modifications and alterations in so far as they come within the scope of the appended claims or the equivalents thereof.

## Claims

1. A device for administering a dose of nutritional supplements, the device comprising:
a fabric layer having an inner surface and an outer surface;
a transdermally diffusible nutritional supplement deposited on the fabric layer, wherein the transdermally diffusible nutritional supplement comprises a mixture of forskohlin, caffeine, and chromium, and wherein the transdermally diffusible nutritional supplement has the property of being diffusible through the skin of an associated user;
an adhesive deposited on the inner surface of the fabric layer for removably affixing the fabric layer to the skin of an associated user.

2. The device of claim 1, wherein the transdermally diffusible nutritional supplement further comprises:
5-hydroxytryptophan; and
diiodotyrosine.

3. The device of claim 2, wherein the transdermally diffusible nutritional supplement further comprises:
a permeation enhancer selected from the group consisting of straight-chain or branched-chain fatty alcohols, including decanol, dodecanol, 2-hexyl decanol, 2-octyl dodecanol and oleyl alcohol; saturated or unsaturated fatty acids, including undecylenic acid, lauric acid, myristic acid and oleic acid; fatty acid esters or their derivatives; fatty alcohol ethoxylates including the reaction products of dodecanol or oleyl alcohol; methyl laurate, ethyl oleate, isopropyl myristate and isopropyl palmitate; water-miscible enhancers including 1,2-propylene glycol, glycerol, 1,3-butanediol, dipropylene glycol and polyethylene glycols; piperine or tetrahydropiperine; and blends thereof; and
at least a first antioxidant selected from the group consisting of epicathechin and proanthocyanidins.

4. The device of claim 3, wherein the permeation enhancer is tetrahydropiperine.

5. The device of claim 4, further comprising:
a release liner removably affixed adjacent the inner surface of the fabric layer.

6. The device of claim 5, wherein the fabric layer is a polyester fabric layer, and wherein the release liner is a mylar release liner.

7. A transdermally diffusible nutritional supplement comprising:
a mixture of two or more supplements selected from a group consisting of a supplement selected to suppress appetite, a supplement selected to increase energy, a supplement selected to support pancreatic function, a supplement selected to support dopamine production, a supplement selected to support thyroid function, a supplement selected to support preservation of lean body mass during reduced caloric intake, and an antioxidant; and
wherein the mixture has the property of being diffusible through the skin of an associated user.

8. The nutritional supplement of claim 7, wherein the supplement comprises:
the supplement selected to suppress appetite, wherein the supplement selected to suppress appetite is a 5-hydroxytrptophan supplement;
the supplement selected to increase energy, wherein the supplement selected to increase energy is a caffeine supplement;
the supplement selected to support pancreatic function, wherein the supplement selected to support pancreatic function is a chromium supplement;
the supplement selected to support thyroid function, wherein the supplement selected to support thyroid function is a diiodotyrosine supplement; and
the supplement selected to support preservation of lean body mass during reduced caloric intake, wherein the supplement selected to support preservation of lean body mass during reduced caloric intake is a forskohlin supplement;

9. The nutritional supplement of claim 8 further comprising:
a permeation enhancer, wherein the permeation enhancer is tetrahydropiperine.

10. A method of administering nutritional supplements comprising the steps of:
providing a patch, wherein the patch is comprised of
a fabric layer having inner and outer surfaces,
a nutritional supplement deposited on the fabric layer, wherein the nutritional supplement is transdermally diffusible through the skin of an associated user,
a release liner removably affixed adjacent the inner surface of the fabric layer, and
an adhesive deposited on the inner surface of the fabric layer;
removing the release liner from the fabric layer; and
removably affixing the inner surface of the fabric layer adjacent the skin of the associated user by means of the adhesive.

11. The method of claim 10, wherein the nutritional supplement is selected for promoting weight loss.

12. The method of claim 11, wherein the nutritional supplement comprises a mixture of forskohlin, caffeine, and chromium.

13. The method of claim 12, wherein the nutritional supplement further comprises:
5-hydroxytryptophan;
diiodotyrosine; and
a permeation enhancer.

14. The method of claim 13, wherein the nutritional supplement further comprises:
at least a first antioxidant selected from the group consisting of epicathechin and proanthocyanidins.

15. Method of claim 12, further comprising the steps of:
leaving the fabric layer on the skin for a first interval of time, wherein the first interval of time is selected to allow a dose of the nutritional supplement to diffuse through the skin of the associated individual;
removing the fabric layer from the skin after the first interval of time has laspsed;
placing a second fabric layer on the skin of the individual for a second interval of time, wherein the second interval of time is selected to allow the dose of the nutritional supplement to diffuse through the skin of the associated individual ;
removing the second fabric layer after the second interval of time has lapsed, and
repeatedly placing and removing subsequent fabric layers until desired weight loss has been attained.
